# EUROPEAN PATENT SPECIFICATION

(11) **EP 1 691 628 B1**
(45) Date of publication and mention of the grant of the patent: **15.07.2009**
(21) Application number: 04803197.5
(22) Date of filing: 19.11.2004
(51) Int. Cl.: A23L 1/30, A23L 1/03, A23L 1/308, C12N 9/10, C12R 1/225

(54) **PROCESS FOR THE FERMENTATIVE ENRICHMENT OF FOODS WITH FRUCTO-OLIGOSACCHARIDES**
VERFAHREN FÜR DIE FERMENTATIVE ANREICHERUNG VON NAHRUNGSMITTELN MIT FRUCTO-OLIGOSACCHARIDEN
PROCEDE D'ENRICHISSEMENT D'ALIMENTS PAR FERMENTATION AU MOYEN DE FRUCTO-OLIGOSACCHARIDES

(30) Priority: 27.11.2003 DE 10355302
(43) Date of publication of application: 23.08.2006
(73) Proprietor: Technische Universität München, 80290 München (DE)
(72) Inventor: GÄNZLE, Michael, Edmonton, AB T6R2P5 (CA); TIEKING, Markus, 67304 Eisenberg-Steinborn (DE); VOGEL, Rudi, 85414 Kirchdorf (DE)
(74) Representative: Fleuchaus, Andrea
(86) International application number: PCT/EP2004/013181
(87) International publication number: WO 2005/051102

(56) References cited:
- WO-A-01/90319
- WO-A-02/50311
- KORAKLI MAHER ET AL: "Exopolysaccharide and kestose production by Lactobacillus sanfranciscensis LTH2590." APPLIED AND ENVIRONMENTAL MICROBIOLOGY, vol. 69, no. 4, April 2003 (2003-04), pages 2073-2079, XP002324282 ISSN: 0099-2240 cited in the application
- DATABASE BIOSIS [Online] BIOSCIENCES INFORMATION SERVICE, PHILADELPHIA, PA, US; 1994, CRITTENDEN R G ET AL: "Identification and characterisation of the extracellular sucrases of Zymomonas mobilis UQM 2716 (ATCC 39676)" XP002324283 Database accession no. PREV199497358187 & APPLIED MICROBIOLOGY AND BIOTECHNOLOGY, vol. 41, no. 3, 1994, pages 302-308, ISSN: 0175-7598
- DATABASE WPI Section Ch, Week 199329 Derwent Publications Ltd., London, GB; Class D16, AN 1993-231471 XP002324284 & JP 05 153952 A (GUNEI KAGAKU KOGYO KK) 22 June 1993 (1993-06-22)

## Description

The present invention refers to a process of directly increasing the amount of fructo-oligosaccharides in foods, wherein firstly the sucrose content in the food is adjusted to a certain concentration and secondly said sucrose is fermented by lactobacilli under defined conditions to fructo-oligosaccharides, primarily to kestose, nystose and also to further hetero-oligosaccharides, primarily containing kestose. The invention further refers to the use of lactobacilli, having a fructosyl transferase activity in the process to increase by fermentation the amount of fructo-oligosaccharides in foods.

A compound with 2 to 10 monosaccharide units, comprising fructose is generally named fructo-oligosaccharide (FOS). In the context of the present invention FOS describes an oligosaccharide with up to 10. FOS comprise for example (i) kestose - a trisaccharide consiting of two fructose molecules and one glucose molecule or (ii) nystose - a tetrasaccharide consisting of three fructose molecules and one glucose molecule. A further oligosaccharide belonging to the FOS as defined herein is the pentasaccharide, kestopentaose, consisting of four fructose molecules and one glucose molecule. Beside glucose, also maltotriose, raffinose, maltose, arabinose and xylose are efficient fructosyl-acceptors and fructo-oligosaccharides comprising these monomers in addition to or alternatively to glucose are called hetero-oligosaccharides (HOS). HOS are also comprised under the definition of FOS according to the present invention.

FOS "primarily consisting of kestose" may contain according to the present invention one molecule of glucose, but also maltotriose, raffinose, maltose, arabinose or xylose and at least two, but also three, four, five, six, seven, eight or nine molecules of fructose.

FOSs are often used as a sugar substitute in foods, because of their low sweetness and the fact that they are essentially calorie-free and noncariogenic. FOS are further used as prebiotic or bifidogenous substance in foods or cosmetic products.

The term "prebiotic substance" as used in the context of this invention, describes compounds, which due to their structure and their increased fructose content will not be degraded by pancreatic enzymes, will not be absorbed in the small intestine and, thus, reach the lower intestine, where they selectively stimulate the growth of bifidobacteria. Therefore, prebiotic substances are also known as "bifidogenous substances". The prebiotic or bifidogenous properties of FOS and particularly of kestose are well-known.

However, in natural food products only minimal amounts of FOS, particularly kestose or oligosaccharides primarily consisting of kestose are found. In most fruits, vegetables or cereals only about 0.1% of the dry substance are FOSs. Kestose or oligosaccharides primarily consisting of kestose have even lower percentages. Only in artichokes, onions and chicory FOS can be found at a percentage of 2-30% of the dry substance.

Currently marketed prebiotic yoghurts or other dairy products generally contain approx. 1% kestose. In order to enrich foods with kestose or other prebiotic sugars, the state of the art only offers chemical or microbiological processes to synthesize and then extract and isolate such FOS, which than can be added to foods.

If one would like, as it is preferred according to the present invention to increase the amount of FOS and particularly kestose or oligosaccharides primarily consisting of kestose in foods to up to an amount of 2-8%, preferably 8-12%, more preferred 12-18% and most preferred 18-25% of FOS, much higher amounts of FOS and particularly kestose or oligosaccharides primarily consisting of kestose are required than can practically be isolated from natural products.

The increased demand for FOS and particularly kestose or oligosaccharides primarily consisting of kestose is currently produced in elaborate microbiologic fermentation and production procedures including additional extraction and purification processes. Currently, three different procedures are being used for the production of FOS.

In a first procedure inulin is processed using the enzyme endo-inulinase. Thus, 2 molecules of sucrose will be enzymatically hydrolyzed to one molecule of kestose and one molecule of glucose. Then, in further steps one molecule of kestose and one molecule of sucrose become one molecule of nystose and one molecule of glucose. Accordingly, the resulting FOS and kestose can be isolated after an extraction step, e.g. a chromatographic purification.

According to a further known process pure FOS, particularly kestose or oligosaccharides primarily consisting of kestose can be enzymatically produced from sucrose in suitable media by using the purified enzyme levansucrase, which is isolated and extracted from bacteria or fungi.

Thirdly, there is a process of fermentative production of FOS, particularly kestose or oligosaccharides primarily consisting of kestose using fungi or bacteria in a sucrose containing substrate combined with extensive purification steps.

The described processes are all cumbersome and cost-intensive due to the complexity of the process itself and the subsequent necessary extraction or purification steps.

It is therefore an object of the present invention to provide an alternative, efficient and low-cost process for the production of FOS, particularly kestose or oligosaccharides primarily consisting of kestose in order to be able to enrich foods or other starting materials with high amounts of FOS.

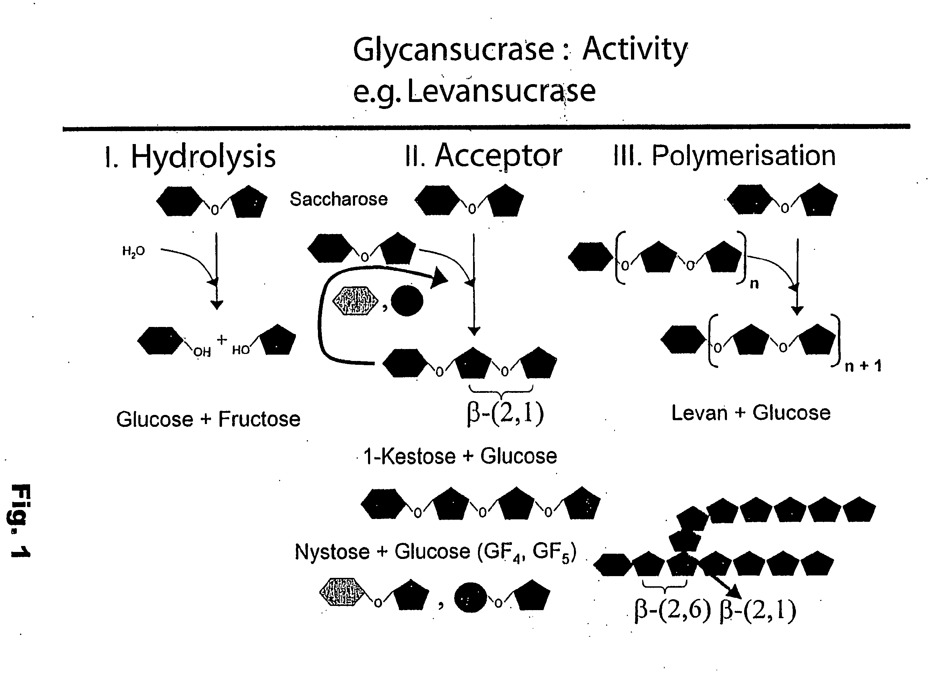

The general synthesis of FOS, particularly kestose or oligosaccharides primarily consisting of kestose, is known for more than 50 years. Moreover, the steps for the enzyme glucansucrase, which synthesizes high molecular oligo- or polysaccharides from sucrose, are well-known. Figure 1 summarizes as example these basic reactions for the enzyme levansucrase. Levansucrase belongs to the family of glycosyltransferase enzymes. These enzymes use the energy of the osidic bond of sucrose to catalyze the transfer of the fructosyl moiety of sucrose to H₂O, an acceptor carbohydrate or a growing levan polymer to yield glucose and fructose, fructo-oligosaccharides and levan.

Further in detail, the levansucrase induces on one hand the hydrolysis of sucrose to glucose and fructose and subsequently catalyses the β(2->1) binding of the accessible fructose molecules. This reaction proceeds until no further sucrose molecule is available. Accordingly, the reaction products are on the one hand low molecular FOSs with two (kestose) or three (nystose) fructose molecules and on the other hand high molecular fructo-polysaccharides with up to n fructose molecules (levan).

Beside this β(2->1) binding the levansucrase on the other hand also catalyzes the β(2->6) linking of long-chain FOS's. This β(2->6) linking is also known as polymerization. in the starting material was adjusted to 5 to 25 w% of the dry substance. These high amounts of sucrose in the starting material favor the enzymatic synthesis of low molecular FOS, preferably the synthesis of kestose or nystose. Advantageously, this reaction produces at the same time reasonable high amount of kestose, before the reaction is slowed down due to the acidification of the starting material caused by the fermentation of lactobacilli.

WO 02/50311 describes already a direct enrichment of foods with levan, a fructo-polysaccharid. Also levan is synthesized by enzymes of lactobacilli having a fructosyl transferase activity, particularly the enzyme levansucrase. Nevertheless, and although a comparable enzyme is involved, the description and particularly the disclosed reaction conditions for fermentation according to the description of WO 02/50311 do not allow the production of kestose in the foods. WO 02/50311 in particular does not teach that high levels of sucrose are needed to switch from the synthesis of levan to the reaction pathway synthesizing low molecular FOS like kestose or nystose.

The inventors of the present invention identified for the first time reasoning for this distinction of the two synthesis pathways. They were able to show that the production of levan takes place in particular when the sucrose content in the foods is around 2% and less of the dry substance. Beside this, it has been surprisingly shown that higher amounts of sucrose inhibit and eventually abolish the production of levan. However, under the same conditions, namely high amounts of sucrose in the starting material the production of kestose and oligosaccharides primarily consisting of kestose is preferred.

Therefore, in the process of the present invention the sucrose content in the starting material has to be adjusted to about 6-25% of the dry substance. In a preferred embodiment the sucrose content is adjusted to 6-15%, further preferred to 8-12% of the dry substance of the starting material.

Further, in the process of the present invention the pH starting range is adjusted to about 8.0-6.0 in order to provide an extended reaction time for the enzymatic kestose production before the acidification due to the fermentation by lactobacilli will stop the whole reaction. The preferred pH range during the kestose production is between about pH 3.5 and pH 7.0.

Furthermore, in the process of the present invention about 10⁶ and 10¹⁰ colony forming units (cfu) of living Lactobacilli having a fructosyl transferase activity will be added to provide for ideal conditions for the kestose production in the starting material. Preferably Therefore, in the process of the present invention the sucrose content in the starting material has to be adjusted to about 6-25% of the dry substance. In a preferred embodiment the sucrose content is adjusted to 6-15%, further preferred to 8-12% of the dry substance of the starting material.

Further, in the process of the present invention the pH starting range is adjusted to about 8.0-6.0 in order to provide an extended reaction time for the enzymatic kestose production before the acidification due to the fermentation by lactobacilli will stop the whole reaction. The preferred pH range during the kestose production is between about pH 3.5 and pH 7.0.

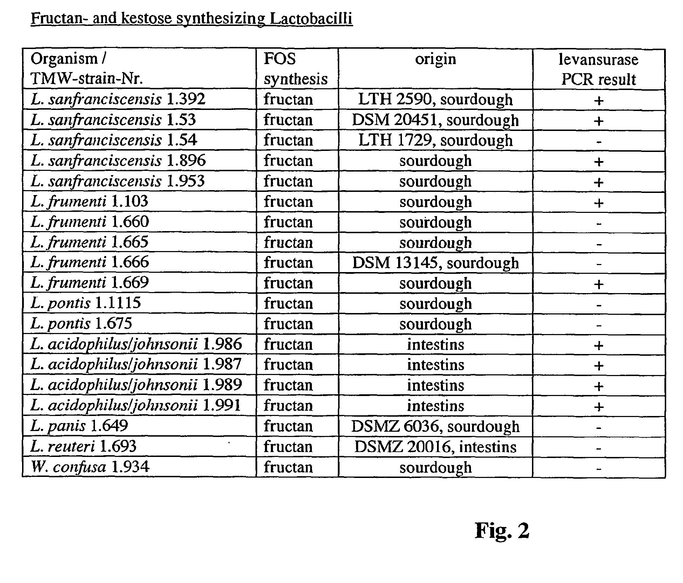

Furthermore, in the process of the present invention about 10⁶ and 10¹⁰ colony forming units (cfu) of living Lactobacilli having a fructosyl transferase activity will be added to provide for ideal conditions for the kestose production in the starting material. Preferably Lactobacilli having the enzyme levansucrase are used in the process of the invention. Further preferred the Lactobacilli as listed in Figure 2, namely *L. sanfranciscensis 1.392, L. sanfranciscensis* 1.53, *L*. *sanfranciscensis* 1.54, *L. sanfranciscensis* 1.896, *L. sanfranciscensis* 1.953, *L*. *frumenti* 1.103, *L. frumenti* 1.660, *L. frumenti* 1.665, *L. frumenti* 1.666, *L. frumenti 1.669, L. pontis* 1.1115, *L. pontis* 1.675, *L. acidophilus*/*johnsonii* 1.986, *L. acidophilus*/*johnsonii* 1.987, *L. acidophilus*/*johnsonii* 1.989, *L*. *acidophi*/*usljohnsonii* 1.991, *L. panis* 1.649, *L. reuteri* 1.693 and *W. confusa* 1.934 are used in the process of the present invention. The numbers behind the name are reference numbers of the Institute of Technical Microbiology Weihnstephan (TMW). All these Lactobacilli have been described previously, they are listed in Figure 2, they all show levansucrase expression and can be requested at the TMW. However even if a practitioner would not be able to get one of these strains, this would not limit the applicability of the invention. Because also further bacterial strains showing a levansucrose expression can be used according to the present invention.

To identify further strains showing a levansucrase expression it is suggested to follow standard procedures and firstly screen the genetic information of different strains by PCR for the presence of levansucrase-specific sequences. Suitable primers for the PCR should be selected to bind to the conserved region of the known sequences of bacterial levansucrase. The PCR is performed under standard conditions as also described in Tieking et al. (2003, Applied and Environmental Microbiology, p. 945-952). PCR amplification results are then analyzed in agarose gel electrophrese and positive bands sequenced to compare them with the known levansucrase sequences, which are for example available in the EMBL-protein and nucleic acid database. To actually proof the expression of levansucrase in the Lactobacilli having a levansucrase gene the RNA of these strains was isolated according to standard procedures in presence and absence of sucrose. Known RT-PCR technology was used to identify levansucrase-specific RNA.

Strains of Lactobacilli, which proofed to be levansucrase positive and are thus suitable for the present invention, can be cultivated in well-known culture media and according to known standard conditions. For example, *L. sanfranciscensis* is cultured in a media at pH 6.2 containing as follows:

| | |
|---|---|
| caseinpepton | 10 g/l; |
| yeast extract | 5 g/l; |
| meat extract | 5 g/l; |
| K₂HPO₄ x 3H₂O | 2.6 g/l; |
| K₂HPO₄ | 4 g/l; |
| Cysteine-HCL | 0.5 g/l; |
| NH₄CL | 3 g/l; |
| Tween 80 | 1 ml/l; |
| MgSO₄ x 3H₂O | 100 mg/l; and |
| MnSO₄ x 4H₂O | 50 mg/l. |

The media is autoclaved at a temperature of 121°C and subsequently 1 ml vitamin stock solution (biotin, folic acid, nicotinic acid, pyridoxal phosphate, thiamine, riboflavine, cobalamine and pantothenic acid 0.2 g/l each) is added. Solutions of maltose, fructose and sucrose will be autoclaved separately and added in concentrations of about 10.5 up to 100 g/l. Cultivation temperature is between 15°C and 45°C, preferred temperature is about 32°C. For cultivation the organisms on solid media controlled incubation conditions like 4% O₂, 20% CO₂, 76% N₂ are used.

For the process of the present invention Lactobacilli having a levansucrase activity and cultivated as described above have been harvested and washed in tab water or physiological saline. The concentration of the washed microorganisms, e.g. *Lactobacillus sanfranciscensis* TMW 1.392, was determined. Preferably, about 10⁶-10¹⁰ cfu (colonie forming units)/ml Lactobacilli were added to the process of the present invention to directly enrich a starting material with FOS, kestose or oligosaccharides primarily consisting of kestose.

Typically the starting materials, foods or cereal products have a pH range between about 3.5 and 8.0. For preferred reaction conditions the pH range is adjusted to a pH of about 6.0 to 8.0 prior to the process.

According the inventive process for direct enrichment of starting materials with FOS the sucrose content in the starting materials has to be adjusted to a sucrose content of about 6 g/kg to 250 g/kg, in other words to about 6-25% of the dry substance. Preferred ranges of sucrose content are also 6-15% or even more preferred 8-12% of the dry substance.

Cereal products, which shall be used for the manufacturing of FOS containing foods, should contain more than 40% water.

The starting materials or foods will be inoculated with the washed lactobacilli and fermented between 12 and 72 hours at about 15-45°C. During this fermentation period FOS and primarily kestose is produced. The fermentation products can be analyzed by HPLC-technology using external standards or enzymatically synthezised standards using purified levansucrase and appropriate acceptor carbohydrates and following standard protocol as previously published, e.g. at Tiele et al. (2002, Analytic Biochemistry 310, p. 171-178).

According to an alternative embodiment instead of living Lactobacilli also inactivated and optionally purified Lactobacilli, which serve only as enzyme source, can be added to the starting material. According to still another embodiment purified enzyme, particularly levansucrase, can be added.

The process according to the present invention is particularly useful to directly enrich starting materials, which can be used for food production, with FOS, kestose and oligosaccharides primarily consisting of kestose. The addition of sucroses to the starting material to allow the production of kestose by levansucrase is exceptionally cost efficient. Furthermore, the inoculation of starting materials with suitable and sufficient Lactobacilli is an easy to handle and cost efficient procedure.

Especially advantageous is the fact that the addition of the various Lactobacilli, which are all regularly used for food manufacturing, and although genetic material of these lactobacilli will remain and can be detected in the final product, this would not lead to additional registration requirements and would not lead to an additional obligation to label the resulting product.

Depending on the selection of starting material individual strains of Lactobacilli can be preferred for using them. For example, *Lactobacillus sanfranciscensis* is particularly preferred for the enrichment of cereal products, namely rye, wheat, barley or oat products (compare to Example 1). For the fermentative enrichment of potato or rice products e.g. *Lactobacilli reuteri* or *frumenti* are preferred.

Eligible starting materials for the process according the present invention are all fruit or vegetable preparations, which can be used to produce or prepare foods, or which can be used as pure or mixed juices, compote, preserve, sauce and pulp, especially also for infant food or dietary products.

Further are milk, dairy products, such as yogurt, curd, cream, butter, spreads and cheese, or milk surrogates (e.g. soy products) suitable starting materials according to the present invention.

Eventually and especially starch containing products, such as substrates made out of cereals like e.g. corn, oat, barley, wheat or rye as well as substrates made out of potatoes, rice or yam are preferred starting materials.

Starch containing substrates are preferably made to a dough or at least mixed with at least 400g/kg or more, in other words 40% or more water.

In summary the process of the present invention is particularly useful for directly enriching foods with FOS, kestose and oligosaccharides primarily consisting of kestose.

Furthermore, the process of the present invention is useful for the direct enrichment of dietary supplements e.g. vitamin preparations. In this case it is for example possible to directly enrich the starch-containing carrier in the dietary supplement with FOS, kestose and oligosaccharides primarily consisting of kestose.

Moreover, the process of the present invention can be used to directly enrich feeds for domestic or farm animals with FOS, kestose and oligosaccharides primarily consisting of kestose.

Finally, the process of the present invention is also useful for the direct enrichment of starting materials for the cosmetic industry, particularly starting materials based on cacao butter or other natural carriers.

The invention is further illustrated by the following figures and examples, which do not limit in any ways the scope of the claims.

### Short description of the figures

- Figure 1: depicts the known enzymatic steps of the enzyme levansucrase.
- Figure 2: lists different strains of Lactobacilli having either a fructosyltransferase activity or the enzyme levansucrase. The synthesis of FOS was shown by a gel permeation chromatography and subsequent analysis of the monomer composition of a certain oligosaccharide. Additionally, PCR analysis using levansucrase-specific PCR primers was performed.

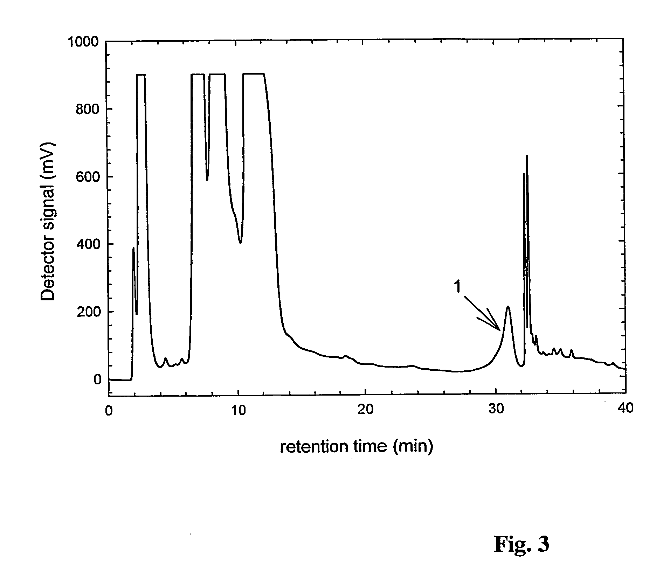

- Figure 3: shows the chromatographic analysis for kestose in fermented apple juice according to Example 1. The peak marked with "1" had been identified as kestose by using external standards. The peaks at 3, 8, 9 and 12 min retention time refer to mannite, glucose, fructose and sucrose.

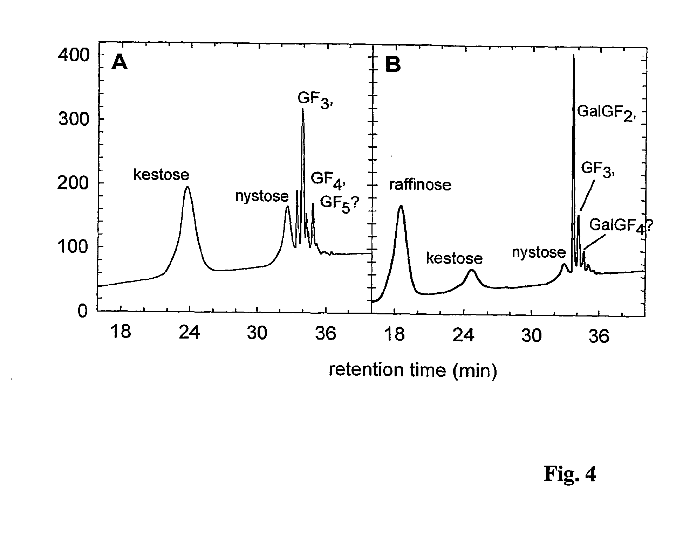

- Figure 4: Separation of oligosaccharides by HPAEC-PAD resulting from the levansucrase reaction with 0.4 Mol L⁻¹ sucrose (PanelA) or raffinose (PanelB). Peaks were assigned based on external standards or literature data on products of levansucrase activity as described in the text. Glucose, fructose, sucrose, and melibiose eluted prior to the retention time cut-off at 16 min.

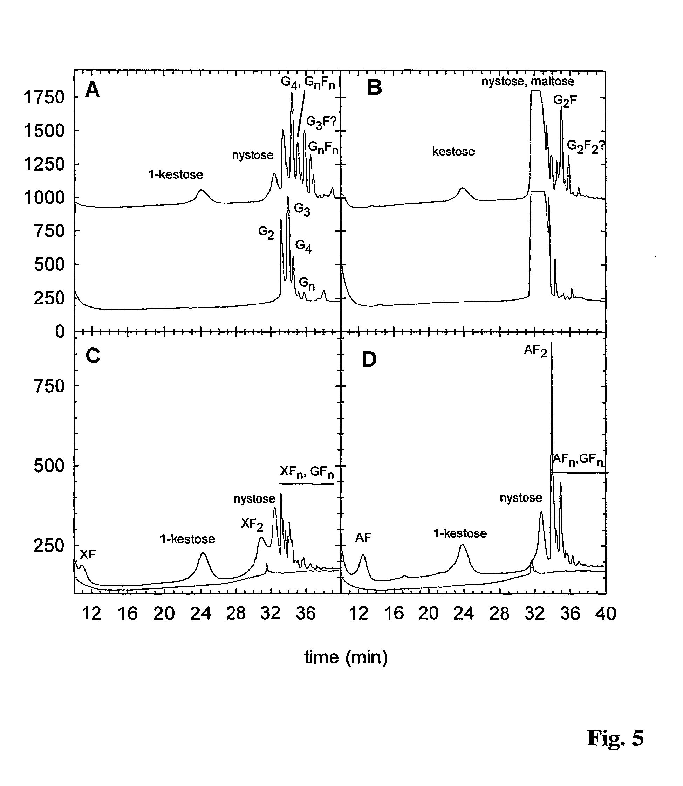

- Figure 5: Separation of oligosaccharides resulting from the levansucrase reaction in the presence of raffinose (A) or sucrose and arabinose (B), followed by two consecutive GPC runs to remove mono-, di-, and trisaccharides. Likewise, oligosaccharides form the maltotriose, maltose and xylose acceptor reactions were prepared (data not shown).

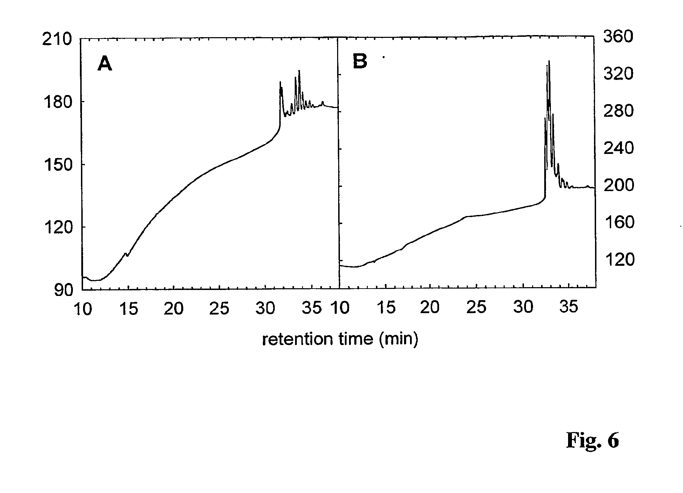

- Figure 6: Separation of oligosaccharides by HPAEC-PAD resulting from the levansucrase reaction with 0.4 Mol L⁻¹ sucrose as fructosyl-donor and 0.4 Mol L⁻¹ maltotriose (A), maltose (B), xylose (C), or arabinose (D) as fructosyl-acceptors (upper traces). The same reaction products were separated after hydrolysis of FOS and HOS by yeast invertase (lower traces). Peaks were assigned based on external standards or literature data on products of levansucrase activity as described in the text. Glucose, fructose, sucrose, xylose and arabinose eluted prior to the retention time cut-off at 16 min used to depict oligosaccharide peaks (not shown).

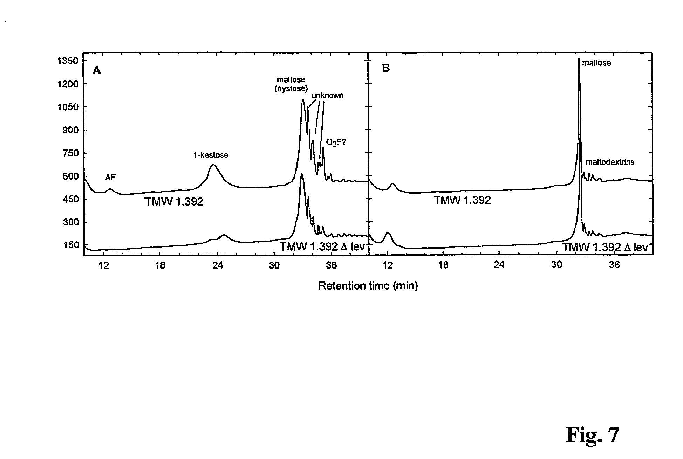

- Figure 7: Separation of oligosaccharides extracted from wheat doughs by HPAEC-PAD. (A) analysis of sourdough aqueous extract fermented with *L. sanfranciscensis* TMW 1.392 (upper trace) or *L. sanfranciscensis* TMW 1.392Δlev (lower trace). (B) analysis of invertase treated sourdough aqueous extracts fermented with *L*. *sanfranciscensis* TMW 1.392 (upper trace) or *L. sanfranciscensis* TMW 1.392Δlev (lower trace). Peaks were assigned based on the comparison of the retention times with external standards, and standards derived from enzymatic reactions (Fig. 6). Mannitol, glucose, fructose, sucrose, and melibiose eluted prior to the retention time cut-off at 10 min (not shown).

### Example 1

### In situ enrichment of foods with kestose

For the *in situ* enrichment of foods with kestose 150 g/l sucrose were mixed into sterile whole milk (pH 6.8), then 10⁸ cfu/g *L. sanfranciscensis* (e.g. strain TMW 1.392) were added and fermentation was allowed for 24 hours at 30°C.

In parallel, apple juice, which had been brought with 2N NaOH to a pH of 6.2, was mixed with 150 g/l sucrose and then was also inoculated with 10⁸ cfu/g *L. sanfranciscensis* (TMW 1.392) and incubated for 24 hours at 30°C.

In a third example, a dough made out of 1000g wheat flour, 1000 g water and 100 g sucrose was inoculated with 10⁷ cfu/g *L. sanfranciscensis* (e.g. strain TMW 1.392) and fermentation was allowed for 24 hours at 30°C.

After the fermentation the samples were centrifuged with 14000 x g for 20 min at room temperature. An aliquot of the supernatant was diluted 1:100 with aqua dest. and chromatographically analyzed by HPLC. The principle of separation is here an ion-exchange chromatography based on an AminoPac PA 10-column and a corresponding guard column (Dionex, Sunnyvale, California). The HPLC system used further comprised a M480 pump, an autosampler Gina 50 and a column thermostat (all components from Gynkothek, Germering, Germany). The detection took place with an ED40 electrochemical detector (Dionex, Bad Idstein) coupled with a pH reference electrode. The chromatographic system control, the data acquisition, and data evaluation was done with Chromeleon 4.1 software.

The injection volume of the samples was 20 µl, the flow rate was 0,25 ml/min and the column had a temperature of 30°C. Probes were eluted with a ternary grandient (solvent A: water; solvent B: 250 mM NaOH; solvent C, 1 M sodium acetate). All solutions were prepared with double demineralized water having a resistance of 18mOhm, which was freshly made at a facility for SG-water (Barsbuettel, Germany). All eluents were exhaustively degassed with helium and kept under slight helium overpressure. Prior to the injection of the sample the columns were washed and equilibrated as follows:
10 min 50% solvent B/50% C; 9 min 100% A; 21 min 88% A/12% C.

After the injection the probe was eluded with the following gradient:
2 min 88% A/12% C; 9 min 90% A/10% C; 9 min 80% A/20% C; 10 min 70% A/30% C; 11 min 44% A/32% B/24% C und 13 min 36% A/40% B und 24% C.

For the identification and quantification of the kestose peak external standards were used. Figure 3 exemplifies a chromatogram for fermented apple juice: kestose is eluted after a retention time of 31 min. Table 1 shows the kestose content in foods before and after a fermentation with *L. sanfranciscensis*.

Besides, kestose also small amounts of other oligosaccharides, primarily containing kestose, e.g. nystose, were detected.

### Example 2

### Characterization of further fermentation products

To fully characterize the products from the levansucrase activity during growth of *L. sanfranciscensis* in sourdough, we aimed to detect HOS originating from the acceptor reaction with those carbohydrates, which are present in substantial levels in wheat and rye doughs. The detection of HOS was achieved on high preformance anion exchance chromatography coupled to pulsed amperometric detection (HPAEC-PAD). Because external standards for the HOS originating from the levansucrase reaction with raffinose, maltotriose, maltose, xylose, and arabinose are unavailable, HOS were synthesized in chemically defined enzymatic reactions with purified levansucrase of *L. sanfrancisensis* in the presence of sucrose as fructosyl-donor and the corresponding carbohydrates as fructosyl-acceptors.

**Strains, plasmids, media and growth conditions.** *L. sanfranciscensis* TMW 1.392 and *L. sanfranciscensis* TMW 1.392Δlev were cultivated anaerobically in modified MRS medium (mMRS, (19)), containing 10 g x L⁻¹ maltose and 5 g x L⁻¹ fructose (mMRS-maltose). The strain *L. sanfranciscensis* TMW 1.392 (isogenic to LTH 2590) harbours a levansucrase gene, *L. sanfranciscensis* TMW 1.392Δlev is an isogenic, levansucrase-negative deletion mutant derivative of *L*. *sanfranciscensis* TMW 1.392 (7). *Escherichia coli* strain JM109 DE3 carrying the plasmid pLEV1 was cultivated aerobically in Luria-Bertani (LB) medium containing 100 µg x L⁻¹ ampicillin at 37 °C. The plasmid pLEV1 is based on the expression vector pET3a and harbours the structural gene for the *L*. *sanfranciscensis* levansucrase downstream of the inducible lacZ promotor and in frame upstream of a 6 x histidin-tag to obtain C-terminally his-tagged proteins (7). Expression of the levansucrase by *E. coli* JM109 DE3 was induced by adding 40 µg x L⁻¹ isopropyl-β-D-thiogalactopyranosid (IPTG) to exponentially growing cultures.

**Purification of the *L. sanfranciscensis* levansucrase and enzymatic synthesis of oligosaccharides.** The levansucrase of *L. sanfranciscensis* TMW 1.392 was purified to homogeneity after heterologous expression in *E. coli* JM109 as described (7). *E. coli* JM109 carrying pLEV1 was grown to an optical density (590 nm) of 0.6, IPTG was added, and cells were further incubated at 30 °C for 12 h. Cells were harvested, washed with 50 ml of binding buffer A (50 mM NaH₂PO₄, 10 mM imidazole, 300 mM NaCl) and broken by ultrasonification (5 x 30 sec with 50% cycle, 90% power). Cell debris was removed by centrifugation (10000 x g, 10 min, 4°C) and the supernatant were applied to a HiTrap chelating nickel-column (Amersham Biosciences, Freiburg, Germany) with a flow of 1.5 mL x min⁻¹. The column was washed with 5.0 ml of 20 % buffer B (50 mM NaH₂PO₄, 300 mM imidazole and 300 mM NaCl) and the his-tagged levansucrase was eluted with a linear gradient from 20 % buffer B to 100 % buffer B. Fractions containing levansucrase were dialyzed against 25 mM sodium acetate buffer with pH 5.4. It was verified by SDS-polyacrylamide gel electrophoresis using an mini-protean electrophoresis cell (BioRad, Laboratories, Hercules, USA) with the Laemmly buffer system according to the instructions of the supplier that the levansucrase was not contaminated by other proteins (data not shown). The protein concentration of the stock solution with the purified levansucrase enzyme was determined with the Bradford method, using the BioRad Protein Assay (BioRad, Laboratories, Hercules, USA) and bovine serum albumine as external standard.

Enzymatic reactions were carried out at 37°C in 10 mM Na-acetate buffer pH 5.4 with an enzyme concentration of 0.5 µg x mL⁻¹, containing 1 mM L⁻¹ CaCl₂, 0.4 Mol L⁻¹ sucrose or 0.4 Mol L⁻¹ raffinose, and 0.4 Mol L⁻¹ acceptor carbohydrate where indicated. The reaction mixture was incubated for 20h and stored frozen for further analysis.

**Partial purification and characterization of oligosaccharides from enzymatic reactions.** Enzymatic reactions were carried out in 1 ml volume with levansucrase using sucrose and maltose, maltotriose, xylose, and arabinose as acceptor carbonydrates. After overnight incubation, the reaction mixture was separated on a Superdex Peptide gel permeation chromatography (GPC) column (Amersham Pharmacia Biotech, Freiburg, Germany) eluted with deionized water at a flow of 0.4 ml min⁻¹. Carbohydrates were detected with an refractive index (RI) detector. In order to estimate the degree of polymerisation (DP) of the oligosaccharides, the column was calibrated using glucose, sucrose, raffinose, and stachyose as molecular weight standards. Peaks corresponding to tri, tetra-or pentasaccharides were collected, pooled, and dried under vacuum. When maltotriose or raffinose was used as acceptor carbohydrate, peaks corresponding to tetra- and pentasaccharides were collected. The dried oligosaccharides were redissolved in 0.5 mL deionized water and subjected to a second chromatographic separation on the same column. Peaks corresponding to oligosaccharides with a DP of (3 or 4) and (4 or 5) were pooled, dried, and redissolved in 0.2 mL deionized water. These fractions were analyzed for the oligosaccharide composition by HPAEC-PAD as described below and it was verified that no contaminating mono- di- or trisaccharides were present (data not shown). Furthermore, oligosaccharides in these fractions were hydrolyzed by incubation for 1h with 5% perchloric acid at 90°C. The hydrolysates were neutralised by addition of 4M KOH. Monosaccharides in the hydrolysates were quantified by HPLC using the CHPB^{®} column (Merck, Darmstadt, Germany) eluted with deionized water at a flow of 0.4 ml min⁻¹ and RI detection as described (16).

**Preparation of doughs, determination of pH and cell counts in sourdough.** Wheat doughs were fermented with *L. sanfranciscensis* TMW 1.392 or the isogenic, levansucrase-negative deletion mutant, *L. sanfranciscensis* TMW 1.392Δlev. For inoculation of doughs, about 10⁹ cells from an overnight culture in mMRS were washed with sterile water and resuspended in 10 mL of tap water. Doughs were prepared with 100 g wheat flour type 550, 90 g sterile tap water, 10 mL cells of strain TMW 1.392 or TMW 1.392Δlev, and 20 g sucrose. The determination of pH-values and cell counts was carried out as described (20) to verify that the strains used to inoculate the doughs dominated the fermentation throughout the incubation time of 24h. For oligosaccharide analysis, dough samples were weighed, diluted with demineralized water and centrifuged at 17000 x g for 30 min to remove solids. These supernatants were diluted 1:100 and used for oligosaccharide analysis as described below.

**Chromatographic separation of oligosaccharides.** The separation of oligosaccharides was achieved by high-performance anion-exchange chromatrography with integrated pulsed amperometric detection (HPAEC-IPAD) using an AminoPac PA10 column and an ED40 electrochemical detector as described (21). A ternary gradient was employed using water with a resistance of 18 MOhm or more (solvent A), 0.25 M NaOH (Solvent B) and 1 M sodium acetate (solvent C). For improved separation of oligosaccharides, the gradient was modified as follows: 0 min, 0% B and 12%C; 2 min, 10% B and 12%C; 11 min, 20% B and 12%C; 18 min, 0% B and 30%C; 36 min, 36% B and 24%C; 45 min, 40% B and 24%C. The column was washed with 50% B and 50% C for 20 min after each sample. Maltose, maltotriose, raffinose, melibiose, and mixtures of inulin-type FOS (Actilight, degree of polymerisation, DP, 2 to 8, Gewürzmüller, Stuttgart, Germany) or maltose-oligosaccharides (DP4 -10, Sigma, Deisenhofen, Germany) were used as external standards. Invertase treatment of enzymatic reactions and aqueous dough extracts were carried out by incubation with yeast invertase at a concentration of 100 µg L⁻¹ for 3h at 37°C.

**Reproducibility.** All enzymatic reactions with levansucrase, and sourdough fermentations with *L. sanfranciscensis* strains were carried out at least in duplicate with consistent results. The retention times of the individual peaks in HPAEC-PAD analysis were reproducible with an error of ± 0.5 min, and peak areas were generally reproducible with an experimental error of less than 15%.

**Enzymatic synthesis of FOS and HOS.** In order to determine the retention times of HOS on the HPAEC-PAD system, HOS were synthesized in enzymatic reactions with purified levansucrase using sucrose as fructosyl-donor and the corresponding carbohydrates as fructosyl-acceptors. Although the elution order of hetergenous sugar oligomers on HPAEC-PAD can not be predicted, oligomers of a homologous series differing only in their DP generally elute in the order of their molecular weight. Our analytical setup allowed the separation of maltose-oligosaccharides and inulin-type FOS up to a DP of 8, however, sufficient separation of HOS with a DP of 4 or more was not achieved in those cases where oligomers of two or more homologous series were present. Therefore, peaks were assigned to HOS based on the following considerations. (i) comparison of the retention times to FOS external standards. (ii) Assignment of additional peaks in reactions with fructosyl-acceptors to HOS known to result from the (repetitive) acceptor reaction of levansucrase with maltotriose, maltose, xylose, and arabinose, which allowed identification of oligomers with a DP of 3 or less. (iii) preparation of tetra-, penta- and hexasaccharides in two consecutive GPC runs, followed by HPAEC-PAD analysis of the oligosaccharides, and the determination of the monomer composition of oligosaccharides.

**FOS and HOS from sucrose or raffinose as fructosyl-donor and -acceptor.** Levansucrase activity with sucrose as the only substrate is known to yield inulin-type FOS with a DP of up to 5. The reaction of the *L. sanfranciscensis* levansucrase with 0.4 M sucrose as fructosyldonor and -acceptor yielded 1-kestose (GF₂), nystose (GF₃) and FOS with a DP of 5 or greater (GF₄ and GF₅), which were identified using FOS as external standards (Fig. 4A).

In reactions of levansucrase with raffinose as fructosyl-donor and -acceptor, melibiose and 1^{F}-β-fructosylraffinose (GalGF₂) is formed (9). In the reaction of the *L. sanfranciscensis* levansucrase with 0.4 M raffinose as fructosyl-donor, peaks corresponding to tetra-, penta-, and hexasaccharides were present in addition to melibiose, raffinose, kestose, and nystose. The major oligosaccharide peak at 34 min was assigned to GalGF₂. HOS from this reaction were prepared by two consecutive runs on a GPC column and collection of peaks corresponding to tetra- and pentasaccharides. Analysis of this oligosaccharide preparation by HPAEC-PAD demonstrated that the preparation was free of oligomers with a DP of 3 or less, nystose was not present and GalGF₂ was depleted (Figure 5A). The molar monomer ratio in this oligosaccharide preparation upon hydrolysis was Gal:Glu:Fru 1:2:5, which demonstrates that raffinose acts as fructosyl-acceptor to yield oligosaccharides with a DP of 4 - 5.

**HOS from maltose and maltotriose as fructosyl-acceptors.** The levansucrase reaction with sucrose as fructosyl-donor and maltotriose or maltose as acceptor yields 1^{F}-β-fructofuranosylmaltotriose (G₃F) or 1^{F}-β-fructofuranosylmaltose (G₂F), respectively, in addition to the FOS-series (12). In the reaction of the *L. sanfranciscensis* levansucrase with 0.4 M sucrose and 0.4 M maltotriose or maltose were present the FOS kestose and nystose (Fig. 6A and 6B). The major HOS in reactions with maltose eluting at 35 min was attributable to G₂F, oligomers with a DP or 4 or greater that were hydrolyzed by invertase were additionally present (Fig. 6B). The unambiguous assignment of HOS from the maltotriose-acceptor reaction was not possible because three homologous series of oligosaccharides were present, maltooligosaccharides, FOS resulting from the acceptor-reaction with sucrose, and additionally the oligosaccharides G₃F, G₃F₂ and G₃F₃ resulting from the acceptor-reaction with maltotriose (Figure 6A). This complex mixture of oligomers was not sufficiently separated with our analytical setup. However, all oligomers from the maltotriose-acceptor reaction eluting after 35 min were hydrolyzed by invertase treatment (Fig.6) and can not be assigned to FOS (compare to Fig 4A), indicating the presence of G₃Fₓ HOS. Accordingly, in the monomers from pentasaccharides prepared by GPC, a glucose to fructose ratio of 2.5:1 was determined. Because the maltotriose preparation used in our experiments contained trace amounts of maltose and maltotetraose (Fig. 6A, lower trace), additional oligosaccharides from maltose and maltotetraose may be present in the reaction products.

**HOS from xylose or arabinose as fructosyl-acceptors.** From the levansucrase reaction in the presence of xylose and arabinose, the products xylsucrose (xylosyl-β-fructofuranoside, XF), 1^{F}-β-fructosyl-xylsucrose (XF₂), and di- and trifructosyl-xylsucrose are obtained, from arabinose was characterized arabsucrose (arabinosyl-β-fructofuranoside, AF) (9, 12, 13, 22). In reactions of the *L. sanfranciscensis* levansucrase with xylose and arabinose, xylsucrose and arabinosucrose were present in substantial amounts (Figures 6C and 6D), and the major peaks at 31 and 34 min are attributable to XF₂ and AF₂, respectively. The presence of HOS resulting from the acceptor-reaction with xylose and arabinose was substantiated by preparation of oligosaccharides with a DP of 3 or more via GPS (Figure 5B and data not shown) and analysis of their monomer composition.

The ratio of glucose: pentose: fructose in tri, tetra- and pentasaccharides from reactions with xylose and arabinose were 3:1:5.5 and 2:1:4, respectively, demonstrating the presence of oligosaccharides with a DP of 3 or greater in enzymatic reactions with xylose and arabinose as fructosyl-acceptors.

**Oligosaccharides formed during sourdough fermentations with *L*. *sanfranciscensis*.** To determine whether HOS are formed during growth of *L*. *sanfranciscensis* TMW 1.392, fermentations in wheat dough were carried out in the presence of 10% sucrose. Doughs fermented with *L. sanfranciscensis* TMW 1.392 were compared to doughs fermented with an isogenic levansucrase-negative derivative of this strain, *L. sanfranciscensis* TMW1.392Δlev. Moreover, aqueous extracts of wheat doughs were compared to aqueous extracts treated with yeast invertase to eliminate fructose-oligosaccharides originating either from the levansucrase reaction, or from flour. The chromatograms of dough extracts and invertase treated dough extracts are shown in Figure 4. Major peaks that were present in the doughs fermented with *L. sanfranciscensis* TMW 1.392, but not in doughs fermented with *L. sanfranciscensis* TMW 1.392Δlev eluted at 12.5, 23.5, 34.0, 34.75 and 35.25 min (Fig. 7A). Chromatograms of extracts from doughs fermented with TMW 1.392 and TMW 1.392Δlev were virtually identical after treatment with invertase, indicating that all these peaks are indeed attributable to FOS or HOS (Fig. 7B). 1-Kestose and AF can be assigned based on external standards and the retention times of the respective compounds derived from the enzymatic reactions, respectively (Fig. 6D). The peak eluting at 35.35 min may correspond to G₂F, AFₙ or G₃F originating from the acceptor-reaction with maltose, arabinose, or maltotriose (Figs. 6A, 6B, 6C). Because maltose is by far the most abundant carbohydrate in sourdough, the formation of G₂F is more likely than that of other HOS. Low amounts of 1-kestose originating from flour were also present in doughs fermented with *L. sanfranciscensis* TMW 1.392Δlev, nystose co-elutes with maltose and can therefore not be identified unambiguously. FOS or HOS eluting between 33.5 and 34.75 min could not be assigned to individual compounds because several FOS and HOS with DP > 3 eluting in that range were generated in enzymatic reactions. Invertase treatments of extracts from doughs fermented with strains TMW 1.392Δlev or TMW 1.392 in the presence of sucrose generated an oligosaccharide eluting at 12 min. This peak was absent in TMW 1.392Δlev fermented doughs but was also observed in dough extracts from chemically acidified doughs when sucrose was present.

Based on the comparison of oligosaccharides present in extracts from doughs fermented with *L. sanfranciscensis* TMW 1.392 and TMW 1.392Δlev, at least five FOS or HOS could be identified which result from levansucrase activity. The formation of 1-kestose was previously reported (7) and evidence for the formation of arabsucrose and 1^{F}-β-fructofuranosyl-maltose was provided in this work. Further HOS in dough remain to be characterized. Maltose is the major carbohydrate in either wheat or rye sourdoughs, and is therefore readily available as fructosyl-acceptor. Substantial levels of arabinose, xylose, and maltodextrins are additionally present *(25, 27)*. Their levels are enhanced by the use of pentosanases and amylases in bread applications (28).

Leavening of bread doughs may be achieved through the metabolic activity of heterofermentative lactic acid bacteria in the absence of yeasts (29), however, in industrial practice, bakers yeast is the leavening agent most commonly used. Because FOS and HOS produced in dough by the *L. sanfranciscensis* levansucrase are hydrolyzed by yeast invertase, these compounds are not likely to be present in bread. Nevertheless, the formation of HOS by *L. sanfranciscensis* needs to be taken into account when sucrose metabolism of this strain is studied to optimize the levan synthesis through the levansucrase activity during growth in sourdough. Moreover, improved knowledge on the formation of FOS and HOS during growth of levansucrase-positive lactobacilli may enable the directed generation of cereal-based, functional ingredients for food and feed use.

**Table 1**

| 1-kestose content (g/kg) | | | |
|---|---|---|---|
| | apple juice | milk | wheat dough |
| Not fermented | 0 | 0 | 0.4 |
| Fermented with *L. sanfranciscensis* | 1.6 | 2.8 | 1.2 |

### References

1. Yun, J. W. Fructooligosaccharides - occurrence, preparation, and application. Enzyme and Microbial Technology 1996, 19, 107-117.
2. Rhee, S.-K.; Song, K.-B.; Kim, C.-H.; Park, B.-S.; Jang, E.-K.; Jang, K.-H. Levan. In Biopolymers; A. Steinbüchel, Baets, S.D., Vandamme, E.H, Ed.; Wiley VCH.: Weinheim, 2002; pp 351-378.
3. Simmering, R.; Blaut, M. Pro- and prebiotics - the tasty guardian angels? Applied Microbiology and Biotechnology 2001, 19-28.
4. Hidaka, H.; Hirayama, M.; Yamada, K. Fructooligosaccharides. Enzymatic preparation and biofunctions. J. Carbohydrate Chemistry 1991, 10, 509-522.
5. van Hijum, S. A.; van Geel-Schutten, G. H.; Rahaoui, H.; van der Maarel, M. J.; Dijkhuizen, L. Characterization of a novel fructosyltransferase from Lactobacillus reuteri that synthesizes high-molecular-weight inulin and inulin oligosaccharides. Appl Environ Microbiol 2002, 68, 4390-8.
6. Korakli, M.; Pavlovic, M.; Ganzle, M. G.; Vogel, R. F. Exopolysaccharide and kestose production by Lactobacillus sanfranciscensis LTH2590. Appl Environ Microbiol 2003, 69, 2073-9.
7. Tieking, M.; Ehrmann, M. A.; Vogel, R. F.; Ganzle, M. G. Molecular and functional characterization of a levansucrase from the sourdough isolate Lactobacillus sanfranciscensis TMW 1.392. Appl. Microbiol. Biotechnol. in press*.*
8. Tieking, M.; Korakli, M.; Ehrmann, M. A.; Ganzle, M. G.; Vogel, R. F. In situ production of exopolysaccharides during sourdough fermentation by cereal and intestinal isolates of lactic acid bacteria. Appl Environ Microbiol 2003, 69, 945-52.
9. Hestrin, S.; Feingold, D. S.; Avigad, G. The mechanism of polysaccharide production from sucrose. 3. Donor-acceptor specificity of levansucrase from Aerobacter levanicum. Biochem J 1956, 64, 340-51.
10. Monsan, P.; Bozonnet, S.; Albenne, C.; Joucla, G.; Willemot, R.-M.; Remaud-Siméon, M. Homopolysaccharides from lactic acid bacteria. International Dairy Journal 2001, 11, 675-685.
11. Feingold, D. S.; Avigad, G.; Hestrin, S. The mechanism of polysaccharide production from sucrose. 4. Isolation and probable structures of oligosaccharides formed from sucrose by a levansucrase system. Biochem J 1956, 64, 351-61.
12. Hestrin, S.; Avigad, G. The mechanism of polysaccharide production from sucrose. 5. Transfer of fructose to C-1 of aldose by levansucrase. Biochem J 1958, 69, 388-98.
13. Tanaka, T.; Yamamoto, S.; Oi, S.; Yamamoto, T. Structures of heterooligosaccharides synthesized by levansucrase. J Biochem (Tokyo) 1981, 90, 521-6.
14. Korakli, M.; Schwarz, E.; Wolf, G.; Hammes, W. P. Production of mannitol by Lactobacillus sanfranciscensis. Advances in Food Sciences 2000, 22, 1-4.
15. Sebastien, V.; Brandt, M.; Cavadini, C.; Hammes, W.; Neeser, J.-R.; Waldbüsser, S. Lactobacillus strain producing levan and its use in human or pet food products. In *International Patent* WO 02/50311 A3*,* 2002.
16. Korakli, M.; Rossmann, A.; Ganzle, M. G.; Vogel, R. F. Sucrose metabolism and exopolysaccharide production in wheat and rye sourdoughs by Lactobacillus sanfranciscensis. J Agric Food Chem 2001, 49, 5194-200.
17. Brandt, M. J.; Münscher, I.; Hammes, W. P. Einfluß von Laktobazillen aus Sauerteigen auf Weizenteigeigenschaften. Getreide Mehl Brot 2003, 57, 15-17.
18. Campbell, J. M.; Bauer, L. L.; Fahey, G. C.; Hogarth, A. J. C. L.; Wolf, B. W.; Hunter, D. E. Selected fructooligosaccharide (1-kestose, nystose, and 1F-b-fructofuranosylnystose) composition of foods and feeds. J Agric Food Chem 1997, 45, 3076-3082.
19. Stolz, P.; Böcker, G.; Hammes, W. P. Utilization of electron acceptors by lactobacilli isolated from sourdough. I. Lactobacillus sanfrancisco. Z Lebensm Unters Forsch 1995, 201, 91-96*.*
20. Thiele, C.; Gänzle, M. G.; Vogel, R. F. Contribution of sourdough lactobacilli, yeast, and cereal enzymes to the generation of amino acids in dough relevant for bread flavor. Cereal Chemistry 2002, 79, 45-51.
21. Thiele, C.; Gänzle, M.; Vogel, R. F. Sample preparation of amino acid determination by integrated pulsed amperometric detection in foods. Analytical Biochemistry 2002, 310, 171-178.
22. Othsuka, K.; Hino, S.; Fukushima, T.; Ozawa, O.; Kanematsu, T.; Uchiuda, T. Characterization of levansucrase from Rhahnella aquatilis JCM-1683. Bioscience Biotechnology Biochemistry 1992, 56, 1373-1377.
23. van Hijum, S. A.; Szalowska, E.; van der Maarel, M. J.; Dijkhuizen, L. Biochemical and molecular characterization of a levansucrase from Lactobacillus reuteri. Microbiology 2004, 150, 621-30.
24. Meng, G.; Fütterer, K. Structural framework of fructosyl transfer in Bacillus subtilis levansucrase. Nature Structural Biology 2003, 10, 935-941.
25. Farine, S.; Versluis, C.; Bonnici, P. J.; Heck, A.; Peschet, J. L.; Puigserver, A.; Biagini, A. Separation and identification of enzymatic sucrose hydrolysis products by high-performance anion-exchange chromatography with pulsed amperometric detection. J. Chromatography A 2001, 920, 299-308.
26. Boskov-Hansen, H.; Andreasen, M. F.; Nielsen, M. M.; Larsen, L. M.; Bach Knudsen, K. E.; Meyer, A. S.; Christensen, L. P.; Hansen, A. Changes in dietary fibre, phenolic acids and activity of endogenous enzymes during rye bread making. Eur Food Res Technol 2002, 214, 33-42.
27. Rouzaud, O.; Martinez-Anaya, M. A. Effect of processing conditions on oligosaccharide profile of wheat sourdoughs. Z Lebensm Unters Forsch 1993, 197, 434-439.
28. di Cagno, R.; de Angelis, M.; Corsetti, A.; Lavermicocca, P.; Arnault, P.; Tossut, P.; Gallo, C.; Gobbetti, M. Interactions between sourdough lactic acid bacteria and exogenous enzymes: effects on the microbial kinetics of acidification and dough textural properties. Food Microbiology 2003, 20, 67-75.
29. Gänzle, M.; Häusle, S.; Hammes, W. P. Wechselwirkungen zwischen Laktobazillen und Hefen des Sauerteiges. Getreide, Mehl und Brot 1997, 51, 209-215.

## Claims

1. An *in situ* process for directly increasing the amount of fructose-oligosaccharides (FOS), kestose and/or oligosaccharides primarily consisting of kestose in a starting material, wherein the starting material is selected from the group of substrates consisting of a fruit substrate, a vegetable substrate, a milk product, a cereal product and mixture thereof comprising:
- adding to the starting material sucrose up to an amount of 6-25 w/% of the dry starting material;
- increasing the water content of the dry starting material to an amount of at least 40 w/%;
- adding 10⁶-10¹⁰ cfu Lactobacilli, which have a fructosyltransferase activity; and
- fermenting the mixture at a temperature of 10-50°C and a pH of 3.5 to 8.5.

2. The process according to claim 1, wherein sucrose is added up to an amount of 6-15 w/% of the dry starting material.

3. The process according to claim 1 or 2, wherein sucrose is added up to an amount of 8-12 w/% of the dry starting material.

4. The process according to anyone of the preceding claims 1 to 3, wherein the Lactobacilli have an active levansucrase enzyme.

5. The process according to anyone of the preceding claims 1 to 4, wherein the Lactobacillus is *L. sanfranciscensis*.

6. The process according to anyone of the preceding claims 1 to 4, wherein the Lactobacilli are selected from the group consisting of *L sanfranciscensis 1.392, L sanfranciscensis* 1.53, *L. sanfranciscensis* 1.54, *L. sanfranciscensis* 1.896, *L. sanfranciscensis* 1.953, *L. frumenti* 1.103, *L. fiumenti* 1.660, *L. frumenti* 1.665, *L. frumenti* 1.666, *L. frumenti* 1.669, *L. pontis* 1.1115, *L. pontis* 1.675, *L. acidophilus*/*johnsonii* 1.986, *L. acidophilus*/*johnsonii* 1.987, *L. acidophilus*/*johnsonii* 1.989, *L. acidopbilus*/*jobnsonii* 1.991, *L. panis* 1.649, *L*. *reuteri* 1.693 and *W. confuse* 1.934.

7. Use of the process according to anyone of the claims 1 to 6 for *in situ* enrichment of FOS, kestose or oligosaccharides primarily consisting of kestose in foods.

8. Use of the process according to anyone of the claims 1 to 6 for *in situ* enrichment of FOS, kestose or oligosaccharides primarily consisting of kestose in dietary supplements.

9. Use of the process according to anyone of the claims 1 to 6 for *in situ* enrichment of FOS, kestose or oligosaccharides primarily consisting of kestose in animal feed.

10. Use of the process according to anyone of the claims 1 to 6 for *in situ* enrichment of FOS, kestose or oligosaccharides primarily consisting of kestose in cosmetic products.

## Patentansprüche

1. *In*-*situ*-Verfahren zum direkten Erhöhen des Anteils von Fructose-Oligosacchariden (FOS), Kestose und/oder Oligosacchariden, die primär aus Kestose bestehen, in einem Ausgangsmaterial, wobei das Ausgangsmaterial aus der Gruppe von Substrate ausgewählt ist, die aus einem Fruchtsubstrat, einem Gemüsesubstrat, einem Milchprodukt, einem Getreideprodukt und einer Mischung daraus besteht, umfassend:
- Hinzufügen von Sucrose zu dem Ausgangsmaterial bis zu einem Anteil von 6 - 25 Gew.-% des trockenen Ausgangsmaterials;
- Erhöhen des Wassergehalts des trockenen Ausgangsmaterials zu einem Anteil von wenigstens 40 Gew.-%;
- Hinzufügen von 10⁶ - 10" KBE Lactobazillen, die eine Fructosyltransferaseaktivität aufweisen; und
- Fermentieren der Mischung bei einer Temperatur von 10 - 50°C und einem pH-Wert von 3,5 bis 8,5.

2. Verfahren gemäß Anspruch 1, wobei Sucrose bis zu einem Anteil von 6 - 15 Gew.-% des trockenen Ausgangsmaterials hinzugefügt wird.

3. Verfahren gemäß Anspruch 1 oder 2, wobei Sucrose bis zu einem Anteil von 8 - 12 Gew.-% des trockenen Ausgangsmaterials hinzugefügt wird.

4. Verfahren gemäß einem der vorhergehenden Ansprüche 1 bis 3, wobei die Lactobazillen ein aktives Lävansucraseenzym aufweisen.

5. Verfahren gemäß einem der vorhergehenden Ansprüche 1 bis 4, wobei der Lactobazillus *L. sanfranciscensis* ist.

6. Verfahren gemäß einem der vorhergehenden Ansprüche 1 bis 4, wobei die Lactobazillen aus der Gruppe ausgewählt sind, die aus *L. sanfranciscensis* 1.392, *L. sanfranciscensis* 1.53, *L. sanfranciscensis* 1.54, *L. sanfranciscensis* 1.896, *L. sanfranciscensis* 1.953, *L. frumenti* 1.103, *L. frumenti* 1.660, *L. frumenti* 1.665, *L. frumenti* 1.666, *L. frumenti* 1.669, *L.pontis* 1.1115, *L. pontis* 1.675, *L. acidopbilus*/*jobnsonii* 1.986, *L. acidophilus*/*johnsonii* 1.987, *L. acidophilus*/*johnsonii* 1.989, *L. acidopbilus*/*jobnsonii* 1.991, *L. panis* 1.649, *L. reuteri* 1.693 und *W. confusa* 1.934 besteht.

7. Verwendung des Verfahrens gemäß einem der Ansprüche 1 bis 6 zur *In-situ-*Anreicherung von FOS, Kestose oder Oligosacchariden, die primär aus Kestose bestehen, in Nahrungsmitteln.

8. Verwendung des Verfahrens gemäß einem der Ansprüche 1 bis 6 zur *In*-*situ-*Anreicherung von FOS, Kestose oder Oligosacehariden, die primär aus Kestose bestehen, in Nahrungsergänzungen.

9. Verwendung des Verfahrens gemäß einem der Ansprüche 1 bis 6 zur *In*-*situ-*Anreicherung von FOS, Kestose oder Oligosacchariden, die primär aus Kestose bestehen, in Tierfutter.

10. Verwendung des Verfahrens gemäß einem der Ansprüche 1 bis 6 zur *In*-*situ-*Anreicherungvon FOS, Kestose oder Oligosacchariden, die primär aus Kestose bestehen, in Kosmetikprodukten.

## Revendications

1. Un procédé in situ pour augmenter directement le montant de fructo-oligosaccharides (FOS), de kestose et/ou d'oligosaccharides constitué primairement de kestose dans un matériau de départ, où le matériau de départ est sélectionné parmi le groupe de substrats constitués d'un substrat de fruit, un substrat de légume, un produit laitier, un produit céréalier et un mélange de ceux-ci comprenant :
- l'ajout au matériau de départ de sucrose jusqu'à un montant de 6-25 % en poids du matériau de départ sec ;
- l'augmentation de la teneur en eau du matériau de départ sec à un montant d'au moins 40 %/ pds ;
- l'ajout 10⁶-10¹⁰ cfu de lactobacilli ayant une activité fructosyltransférase ; et
- la fermentation du mélange à une température de 10-50°C et un pH de 3,5 à 8,5.

2. Le procédé selon la revendication 1, où le sucrose est ajouté jusqu'à un montant de 6-15 % en poids du matériau de départ sec.

3. Le procédé selon la revendication 1 ou 2 où le sucrose est ajouté jusqu'à un montant de 8-12 % en poids du matériau de départ sec.

4. Le procédé selon l'une quelconque des revendications précédentes 1 à 3 où les lactobacillis ont une enzyme levansucrase active.

5. Le procédé selon l'une quelconque des revendications précédentes 1 à 4, où le lactobacilli est L sanfranciscensis.

6. Le procédé selon l'une quelconque des revendications 1 à 4 où les lactobacillis sont sélectionnés parmi le groupe constitué de
L sanfranciscensis 1.392, L sanfranciscensis 1.53, L sanfranciscensis 1.54, L sanfranciscensis 1.896, L sanfranciscensis 1.953, L frumenti 1.103, L frumenti 1.660, L frumenti 1.6665, L frumenti 1.666, L frumenti 1,669, L pontis 1.1115, L pontis 1.675, L acidophilus/johnsonü 1.986, L acidophilus/johnsonü 1.987, L acidophilus/johnsonü 1.989, L acidophilus/johnsonü 1.991, L panis 1.649, L reuteri 1.693 et W confusa 1.934.

7. Utilisation du procédé selon l'une quelconque des revendications 1 à 6 pour un enrichissement in situ de FOS, kcstose ou oligosaccharides principalement constitué de kestose dans l'alimentation.

8. Utilisation du procédé selon l'une quelconque des revendications 1 à 6 pour un enrichissement in situ de FOS kestose ou oligosaccharides constitué principalement de kcstose dans les compléments alimentaires.

9. Utilisation du procédé selon l'une quelconque des revendications 1 à 6 four un enrichissement in situ de FOS kestose ou oligosaccharides constitué principalement de kestose dans l'alimentation animale.

10. Utilisation du procédé selon l'une quelconque des revendications 1 à 6 four un enrichissement in situ de FOS kestose ou oligosaccharides constitué principalement de kestose dans les produits cosmétiques.
